# EUROPEAN PATENT APPLICATION

(11) **EP 2 292 193 A2**
(43) Date of publication of application: **09.03.2011**
(21) Application number: 10175445.5
(22) Date of filing: 06.09.2010
(51) Int. Cl.: A61F 9/00, A61F 9/007, A61M 5/20, A61M 5/145, A61M 5/155, A61M 5/34

(54) **Device for injecting into an eyeball a sterile liquid selected from amongst perfluorocarbons**

(30) Priority: 08.09.2009 IT VR20090134
(71) Applicant: AL.CHI.MI.A. S.r.l., 35020 Ponte S. Nicolò (Padova) (IT)
(72) Inventor: Beccaro, Mauro, 35010, Cadoneghe (Padova) (IT); Bettini, Enrico, 30032 Fiesso d'Artico (Venezia) (IT); Signori, Paolo, 37131 Verona (IT)
(74) Representative: Ponchiroli, Simone

(57) **Abstract**

Device for injecting into an eyeball a sterile liquid selected from amongst perfluorocarbons, comprising a tubular container body (3) having a first dispensing end (5) and a second end (6), and an adapter (4) for connecting a thrust fluid feed tube (6) to the second end (6). A first stopper (17) is inserted into the container body (3) in a sealed fashion and sterile liquid (2) is fed between the first stopper (17) and the first end (5). The adapter (4) consists of first and second connecting means (21), (22) to attach the adapter (4) to a feed tube (20) and to the second end (6), as well as first and second seal means (23), (24) to guarantee the fluid seal between the adapter (4) and the feed tube (20) and the container body (3). A duct (25) for the passage of a thrust fluid extends through the adapter (4) from the feed tube (20) to the container body (3).

## Description

This invention relates to a device for injecting into an eyeball a sterile liquid selected from amongst perfluorocarbons

In particular, this invention refers to a new device that can be used in the surgical treatment of ophthalmic pathologies. Indeed, as described in patents US 5,037,384, US 5,336,175 and US 5,792,099, for example, some ophthalmic pathologies, such as retinal detachments, are treated surgically by injecting a liquid into the eye which acts as a surgical endotamponade. In particular, in the case of treatment for retinal detachment, the liquid injected into the eyeball is chosen from the perfluorocarbon family. This liquid wholly or partly replaces the vitreous humour and thus, thanks to its high surface tension, is able to push against the detached or curled retina, spreading and remaining adherent to the inner wall of the eyeball to enable subsequent reattachment.

In accordance with consolidated practice, insertion of the aforementioned liquid compounds into the rear chamber is generally performed using a normal syringe which, following extraction from its packet (whose purpose is to keep the syringe sterile), is initially coupled with a needle. The surgeon then uses the syringe to make a hole in the rubber seal of the container of the sterile liquid compound and aspirates the required amount.

At this point, the needle is removed from the syringe and replaced with a canula for intraocular injection (normally fitted with a small hose coupling to allow the syringe to be kept far enough away from the area being operated on).

Alternatively, this same applicant has designed a package containing a syringe prefilled with the sterile liquid (described in European patent application EP 2 020 245 A1). In this case, the surgeon need do no more than extract the syringe from the package and attach the canula.

In all known forms of implementation, the dispensing of the sterile liquid into the rear chamber is performed manually by pressing the syringe plunger. However, this recognized methodology presents several inconvenient aspects.

Indeed, first of all, in order to be implemented, it always requires the simultaneous intervention of two operators, the surgeon and his/her assistant.

Indeed, at the moment in which the perfluorinated liquid is injected, both the surgeon's hands are occupied: with one, he/she is holding the end of the canula dispensing the perfluorinated liquid or the syringe itself, while with the other, he/she is holding other surgical instruments (which vary from case to case). Consequently, the surgeon is unable to press the syringe plunger at the same time, which, hence, must be done by the assistant.

In the second place, the fact that it is the assistant dispensing the sterile liquid requires extreme coordination between the two operators. Indeed, to perform the operation in the best way possible, the fluid must be dispensed at the right speed according to the position of the canula within the rear chamber and the quantity of gas that is gradually aspirated. The entire operation is made even more difficult by the fact that the volume of the rear chamber is minute and the surgeon must observe through a microscope. Furthermore, owing to the dimensions of the syringe, friction with the stopper as it moves and human limitations, it is almost impossible to guarantee that the sterile liquid is dispensed gradually and uniformly; on the contrary, the sterile liquid is normally dispensed in spurts.

Patent US 5,037,384 describes an alternative methodology where the sterile liquid is held in a containment chamber, basically consisting of a syringe cylinder with no stopper and plunger and closed on the top with a rubber seal. In this case, in order to use the liquid, the surgeon makes a hole in the seal using a needle connected to a pressurised air source and monitors the flow of liquid via a valve located near the dispensing canula (which, in this case, is rigid and has no coupling tubes).

However, this methodology also has its inconvenient aspects. Indeed, by injecting pressurised air directly into the sterile liquid, there is a risk of generating excessive pressure which may cause damage to the eye. Moreover, in this case too, it is difficult to activate the valve in such a way as to obtain a gradual flow.

In this situation, the technical aim behind the present invention is to create a device for injecting a sterile liquid chosen from the perfluorocarbon family into the eyeball which overcomes the aforementioned inconvenient factors. In particular, the technical task of the present invention is to create a device for injecting a sterile liquid chosen from the perfluorocarbon family into the eyeball which can be used directly and autonomously by the surgeon, without the need for external assistance, which, at the same time, enables the gradual flow of the liquid.

Further, it is the technical task of the present invention to create a device injecting a sterile liquid chosen from the perfluorocarbon family into the eyeball which does not expose the patient's eye to unnecessary risks.

The specified technical task and the exposed aims are essentially achieved with a device for injecting a sterile liquid chosen from the perfluorocarbon family into the eyeball as described in the annexed claims.

Further characteristics and advantages of the present invention will appear in greater depth in the detailed description of some of the preferred, but not exclusive, methods of execution of a device for injecting a sterile liquid chosen from the perfluorocarbon family into the eyeball, which are illustrated in the annexed designs, where:

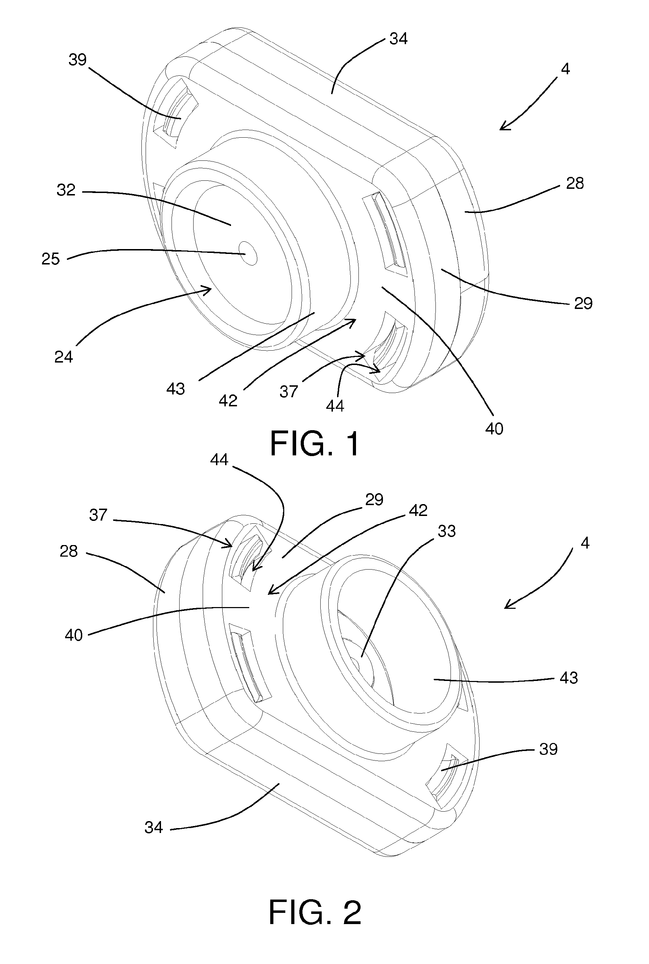

- figure 1 shows an axonometric view of an adapter which is part of the device that is the subject of the present invention;
- figure 2 shows the adapter in figure 1 from a different angle and with no internal stopper;
- figure 3 shows an axial section of a container body which is part of the device that is the subject of the present invention;
- figure 4 shows an axial section of a portion of a tube for feeding pressurised fluid;

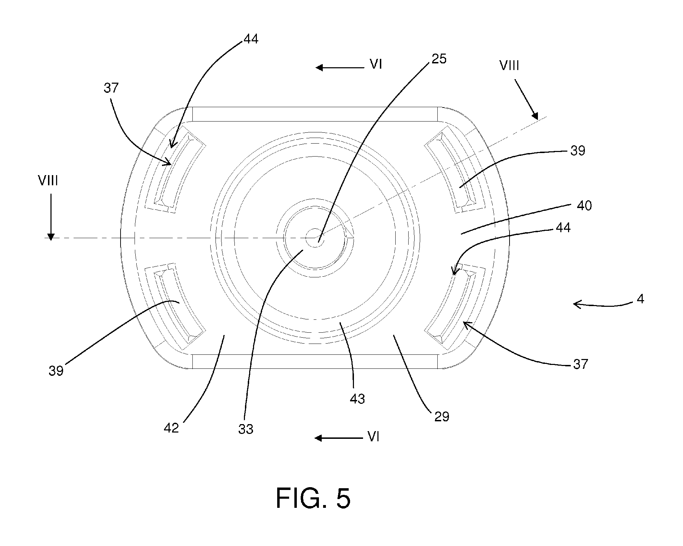

- figure 5 shows the adapter in figure 2 from below;
- figure 6 shows the adapter in figure 5 according to trace VI-VI;
- figure 7 shows the adapter in figure 6 coupled to a second end of the container body in figure 3;
- figure 8 shows the adapter in figure 5 according to trace VIII-VIII;
- figure 9 shows the adapter in figure 8 coupled to the second end of the container body in figure 3;

In its more general design form, the device in the present invention consists of two fundamental elements, a container body 3 which holds the sterile liquid 2, and an adapter 4 which allows the liquid to be connected sealed to a source of pressurised thrust fluid. The sterile liquid 2 is a liquid 2 injectable into the eyeball and is chosen from the perfluorocarbon family.

In particular, the container body 3 is tubular and has a first dispensing end 5 and a second end 6. As can be seen in the annexed figures, the container body 3 advantageously consists of the cylinder of a traditional syringe and advantageously made of glass.

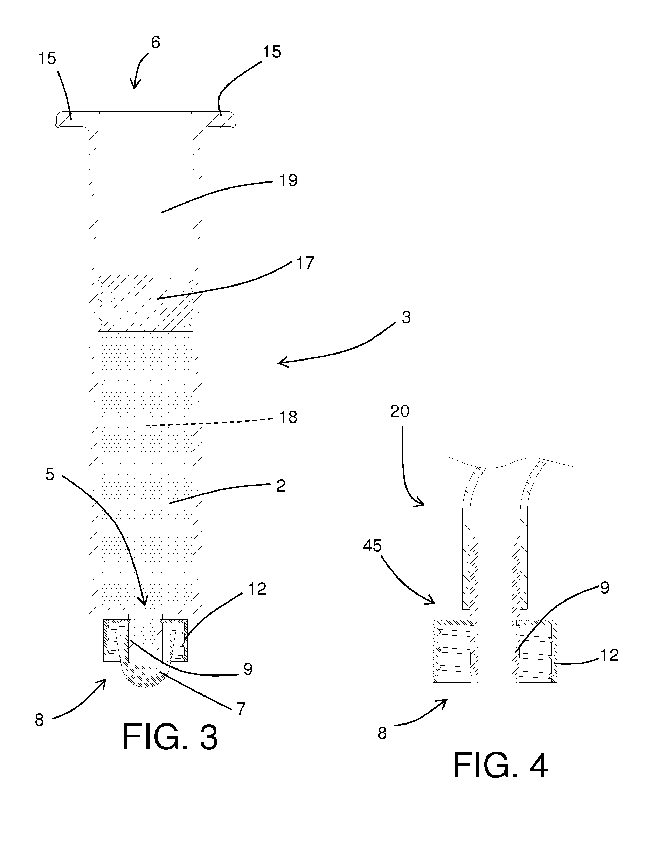

The first end 5 is nozzle-shaped, has a smaller cross-section than the other end and can be connected during use to a dispensing means (such as a canula, not illustrated here as the variety is well known). Before use, the first end 5 is closed by a seal 7. To enable attachment of the dispensing means, the first end 5 may also be fitted with a male or female Luer fitting. Luer fittings are standardised attachments in the medical sector, with a double male-female connector. A first part 8 of the fitting includes a male nozzle 9 which can be inserted in a sealed fashion into a female connector 10 hollowed out from the second part 11; furthermore, to ensure the tightness of the seal, the first part 8 is also fitted with an internally threaded bushing 12 mounted around the nozzle 9, into which a threading can be mounted or plurality of radial teeth 13 shaped from the outside of a coupling 14 which defines the inside of the female connector 10. Examples of Luer attachments can be seen in figures 3 and 4 (two second parts 11 referring to the identification in the previous paragraph) and 6-9 (two first parts 8 referring to the identification in the previous paragraph). Hereafter, male and female Luer fittings will be referred to as the first part 8 and the second part 11 respectively, as defined above (male-female identification is thus made with reference to the fluid duct).

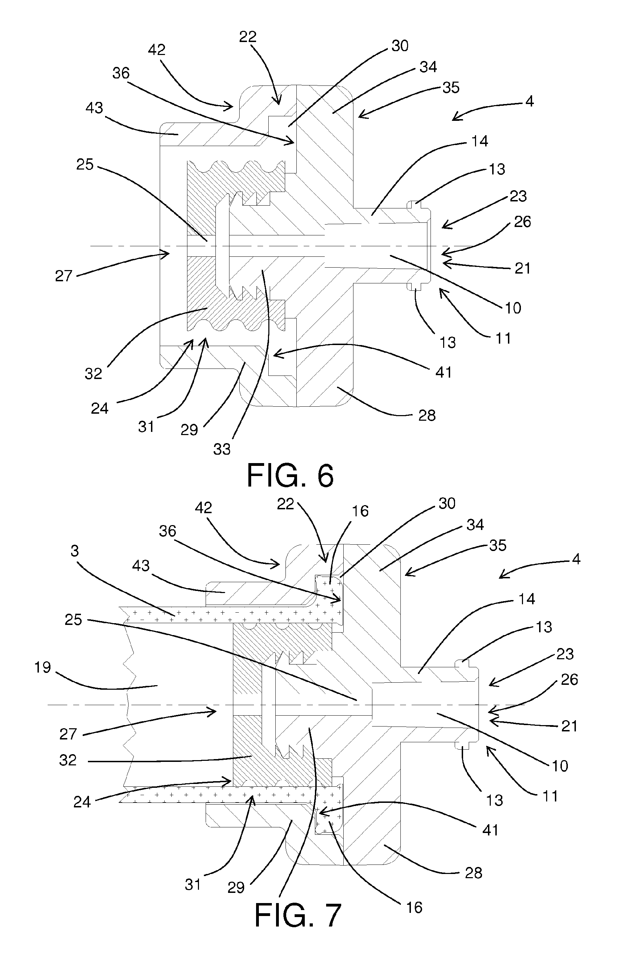

The second end 6 is opposite the first end 5 and its cross-section corresponds essentially to the container body 3 (with the exception of the first end 5). In the design illustrated, moreover, the container body 3 presents a radial projection at the second end 6 consisting of two flat tabs 15 perpendicular to the main axis of the container body 3, diametrically opposite it and coupled via a smaller edge 16 which is level with it (viewed in cross-section in figure 7).

A first stopper 17, made from flexible material is slid opportunely into the container body 3, and opportunely at a distance from the second end 6, (the first stopper 17 should preferably be coated with a flexible, polymer film which is inert to the sterile liquid 2 and opportunely consisting of a fluorinated polymer). The first stopper 17 divides the internal volume of the container body 3 into two chambers, a collection chamber 18 close between the first end 5 and the first stopper 17, filled with sterile liquid 2, and a thrust chamber 19 closed between the first stopper 17 and the second end 6.

The seal 7 of the first end 5 may be opportunely made of the same material as the first stopper 17.

The adapter 4 opportunely has the function of connecting the second end 6 of the container body 3 with a tube 20 for feeding a thrust fluid into the thrust chamber 19.

For this purpose, in general, the adapter 4 includes, first of all, first connecting means 21 to attach the adapter 4 to the feed tube 20 and second connecting means 22 to attach the adapter 4 to the second end 6 of the container body 3, as well as first seal means 23 to guarantee the fluid seal between the feed tube 20 and the adapter 4 and second seal means 24 to guarantee the fluid seal between adapter 4 and the container body 3. Further, the adapter 4 has at least one duct 25 for the passage of a thrust fluid, extending from an infeed section 26 to an outfeed section 27.

The infeed section 26 is positioned for when the adapter 4 is attached to a feed tube 20 by the first connecting means 21; in this position, the infeed section 26 is in fluid connection with to the inside of the tube 20. In turn the outfeed section 27 is positioned for when the adapter 4 is attached to the container body 3 by the second connecting means 22; with this layout, the outfeed section 27 is in fluid connection with the thrust chamber 19 of the container body 3.

This way, the pressurised thrust fluid dispensed through the tube 20 can reach the thrust chamber 19 and create a uniform thrust on the first stopper 17, creating a gradual shift towards the first end 5, with consequent dispensing of the sterile liquid 2.

In the preferred design form, the first connecting means 21 consist of a Luer fitting 11, 12, male or female (female in the annexed figures) designed to connect to a corresponding Luer type connector 11, 12, female or male respective (male in figure 4), attached to the feed tube 20. The first connecting means 21 also consist opportunely of the first seal means 23 (in fact the seal is guaranteed by the tightening of the Luer fitting 11, 12).

In the illustrated design, the second connecting means 22 made by dividing the adapter 4 into a first body 28 and a second body 29 which are hooked onto each other. Once hooked, the first body 28 and the second body 29 form a housing 30 for containing at least the second end 6 of the container body 3 (in other words, the housing 30 forms the second connecting means 22). In the annexed figures, in particular, the housing 30 is shaped to surround, lock and completely contain the radial projection of the container body 3.

The first body 28 can be sealed to the second end 6 of the container body 3. In particular, this can be achieved by attaching the first body 28 to the second end 6 in the same direction as the main axis of the container body 3 itself. In particular, the second seal means 24 consist of a projecting element 31 attached to the first body 28 and made of elastically deformable material (such as rubber), which can be inserted into the second end 6 in a sealed fashion (in this case, the duct 25 extends at least partly through the projecting element 31 itself). In the illustrated design, the projecting element 31 consists of a second stopper 32, similar to the first, mounted onto a rigid support element 33 projecting from and part of the first body 28.

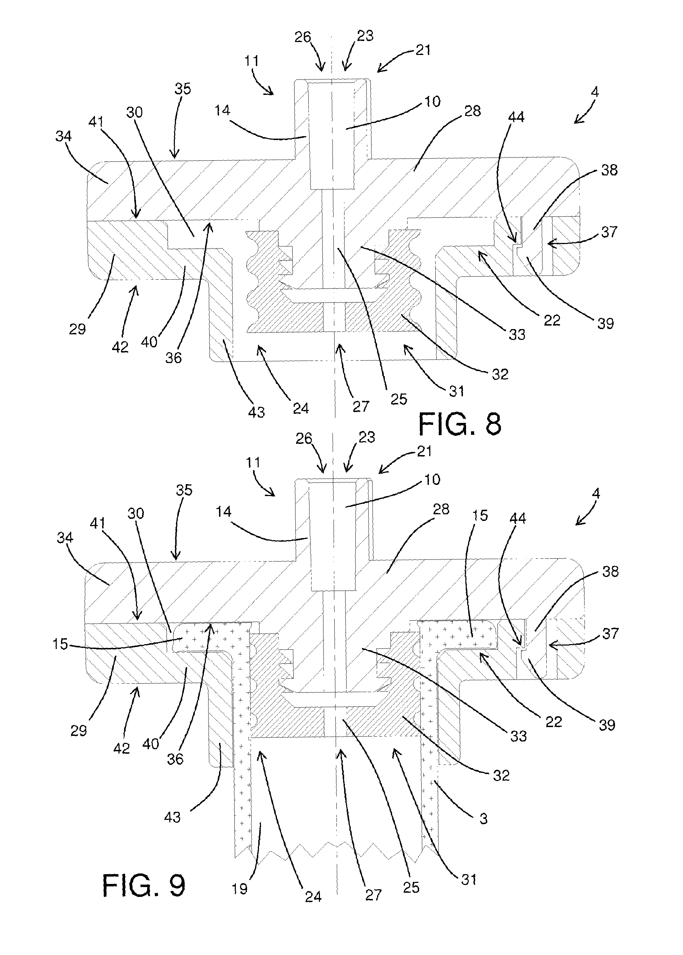

As can be seen in figures 6 and 8, the first body 28 consists of single block of material and includes a first main portion 34 mainly extending flat which has a first 35 on which the first connecting means 21 are mounted (consisting of a female Luer fitting 11, 12), and a second face 36 on which the second seal means 24. Near the edge of the second face 36 a plurality of hooked teeth 37 are mounted. Presented in cross-section, each of these consists of a stem 38 which has an engagement head 39 designed to hook to the second body 29 as explained below.

The second body 29 has an annular shape shaped to match the outside of the container body 3 thus being able to slide along the container body 3 from the first end 5 towards the second end 6 hooking to the first body 28. In particular, the second body 29 consist of a second main portion 40 extending mainly flat (essentially similar to the first main portion 34) and with a third face 41, in practice connectable to the second face 36, and a fourth face 42 opposite the third face, from which there extends cantilever-style a tubular element 43 the inside of which is shaped to match the outer wall of the container body 3, the hole in the tubular element 43 extends as far as the third face 41 so that the second body 29 takes the overall shape of a bushing.

The second 36 and/or the third face 41 (only the latter is shown in the annexed figures) create the housing 30 for the radial projection of the container body 3.

Further, the second main portion 40 also has on the third face 41 undercut seats 44 which can be engaged by the hooking teeth 37. To hook the two bodies 28, 29 together, it is sufficient to push the teeth 37 into the seats 44, deforming them elastically until they are hooked in.

In the illustrated design, the duct 25 is made through the first body 28 (and in particular through the first main portion 34), the rigid support element 33 and the second stopper 32.

In its more complete design, the device that is the subject of the present invention may also include a tube 20 (figure 4) with a first end 45 able to connect to the first connecting means 21 (e.g. it may have special third connecting means such as a Luer fitting 11, 12) and a second end able to connect to a source of pressurised thrust fluid (the second end, not illustrated in the annexed figures, will also be fitted with special connecting means to the source of pressurised thrust fluid).

Lastly, the various components of the device that is the subject of the present invention (container body 3, adapter 4, tube 20, etc...) may be held in one or several sealed container packages (not illustrated) with sterile air. A dispensing canula may also be inserted in these packages to complete the device).

The adapter by itself is part of the present invention 4, provided it is destined to be used with container bodies of the type described above containing a sterile liquid 2 chosen from the family of perfluorocarbons. The device that is the subject of the present invention functions as described above.

In particular, when ready for use, the surgeon removes the components from the package and, connects the adapter 4 to the syringe (if not already connected) and then connects the adapter 4 to the tube 20 and, possibly, connects the tube 20 to the source of pressurised thrust fluid. He or she can then remove the seal 7 of the container body 3 and connect the dispensing canula.

The surgeon may then insert the canula into the eye together with the aspirator (using both hands) and monitor the flow of the liquid 2 via the pedal control which is usually fitted on pressurised fluid equipment.

In the preferred designs of the present invention, the injection pressure supplied by the equipment is set at between 0.1 and 1.5 bars. Indeed, with pressures of between 0.2 and 0.3 bars, flow may be intermittent, while with pressures at 0.7 bars, the liquid flow can form continuously and gradually. The present invention provides important advantages.

Thanks to the possibility of using a control pedal, on one hand, liquid can be dispensed by the surgeon himself/herself; on the other hand, the flow can be monitored with high precision and can take place continuously and gradually with no intermittence, given that the pedal controls the pressure within the thrust chamber.

Furthermore, the solution of injecting the thrust fluid behind the stopper, and not into the liquid, allows avoiding the risk of damaging the eye.

It is also important to note that the present invention is relatively easy to produce and the production costs of the invention are not very high.

The invention design can be subject to numerous modifications and variants, all within the inventive concept which characterises it.

All parts may be replaced with other elements that are technically equivalent, and in practice, all the materials employed, along with the shapes and dimensions of the various components, may be of any nature, according to need.

## Claims

1. A device for injecting into an eyeball a sterile liquid (2) selected from amongst perfluorocarbons, comprising:
a tubular container body (3) having a first, dispensing end (5) with a smaller cross-section which can be connected to dispensing means, and a second end (6) with a larger cross-section which is opposite the first end; and
a first stopper (17) inserted in a sealed fashion in the container body (3);
a quantity of the sterile liquid (2) being inserted in the container body (3) between the first stopper (17) and its first end (5);
and a thrust chamber (19) between the first stopper (17) and the second end (6) of the container body (3);
the device being **characterised in that** it also comprises an adapter (4) for connecting to the container body (3) a tube (20) for feeding a thrust fluid to the thrust chamber (19), the adapter (4) comprising:
first connecting means (21) for attaching the adapter (4) to a feed tube (20);
second connecting means (22) for attaching the adapter (4) to the second end (6) of the container body (3);
at least one duct (25) for the passage of a thrust fluid, extending from an infeed section (26) to an outfeed section (27), the infeed section (26) being positioned in such a way that, when the adapter (4) is attached to a feed tube (20) by the first connecting means (21), the infeed section is in fluid connection with an inner part of the tube (20), and the outfeed section (27) being positioned in such a way that, when the adapter (4) is attached to the container body (3) by the second connecting means (22), the outfeed section is in fluid connection with the thrust chamber (19);
first seal means (23) for guaranteeing the fluid seal between the adapter (4) and the feed tube (20); and
second seal means (24) for guaranteeing the fluid seal between the adapter (4) and the container body (3).

2. The device according to claim 1, **characterised in that** the first connecting means (21) comprise a Luer type connector (11), (12), male or female, designed to connect to a corresponding Luer type connector (11), (12), respectively female or male, attached to the feed tube (20).

3. The device according to claim 1 or 2, **characterised in that** the first connecting means (21) also form the first seal means (23).

4. The device according to any of the foregoing claims, **characterised in that** the adapter (4) comprises a first body (28) and a second body (29) which can be hooked together, the first body (28) and the second body (29), when hooked together, forming a housing (30) for containing at least the second end (6) of the container body (3), said housing (30) forming the second connecting means (22).

5. The device according to claim 4, **characterised in that** the first body (28) can be connected in a sealed fashion to the second end (6) in a direction of movement corresponding to a main axis of extension of the container body (3), and also being **characterised in that** the second body (29) has an annular shape shaped to match the outside of the container body (3) thus being able to slide along the container body (3) from the first end (5) towards the second end (6).

6. The device according to claim 5, **characterised in that** the second seal means (24) comprise a projecting element (31) attached to the first body (28) and made of elastically deformable material, there being the possibility of inserting the projecting element (31) in a sealed fashion in the second end (6) and the duct (25) extending through the projecting element (31).

7. The device according to claim 6, **characterised in that** the projecting element (31) comprises a second stopper (32) mounted on a rigid supporting element (33) integral with the first body (28).

8. The device according to any of the claims from 4 to 7, **characterised in that** the duct (25) is made through the first body (28).

9. The device according to any of the claims from 4 to 8, **characterised in that** the container body (3) comprises at least one radial projection positioned at the second end (6), and also being **characterised in that** the second connecting means (22) act on said radial projection, locking it between the first body (28) and the second body (29).

10. The device according to claim 9, **characterised in that** the first body (28) comprises a first main portion (34) mainly extending flat which has a first face (35) on which the first connecting means (21) are mounted, and a second face (36) on which the second seal means (24) are mounted and a plurality of hooking teeth (37), the duct (25) extending through the first main portion (34), and also being **characterised in that** the second body (29) comprises a second main portion (40) extending mainly flat having a third face (41), in practice connectable to the second face (36), and a fourth face (42) opposite the third face, from which there extends cantilever-style a tubular element (43) the inside of which is shaped to match the outer wall of the container body (3), the hole in the tubular element (43) extending as far as the third face (41), the second and/or third face (41) also having a seat for the housing (30) of the at least one radial projection of the container body (3), and the second main portion (40) also having on the third face (41) undercut seats (44) which can be engaged by the hooking teeth (37).

11. The device according to any of the foregoing claims, **characterised in that** it also comprises a tube (20) having a first end (45) able to connect to the first connecting means (21) and a second end able to connect to a source of pressurised thrust fluid.

12. The device according to any of the foregoing claims, **characterised in that** it also comprises one or more packs for containing the various components.

13. An adapter for connecting a tube (20) to a thrust chamber (19) of a container body (3) having a first, dispensing end (5) with a smaller cross-section able to connect to dispensing means, and a second end (6) with a larger cross-section which is opposite the first end, and a first stopper (17) inserted in a sealed fashion in the body, a quantity of a sterile liquid (2) to be injected into an eyeball selected from amongst perfluorocarbons being inserted in the container body (3) between the first stopper (17) and its first end (5), and there being a thrust chamber (19) between the first stopper (17) and the second end (6) of the container body (3), the adapter being **characterised in that** it is made according to any of the claims from 1 to 10.
